# EUROPÄISCHE PATENTSCHRIFT

(11) **EP 0 814 762 B1**
(45) Veröffentlichungstag und Bekanntmachung des Hinweises auf die Patenterteilung: **28.11.2001**
(21) Anmeldenummer: 96907300.6
(22) Anmeldetag: 22.03.1996
(51) Int. Cl.: A61K 7/48

(54) **KOSMETIKUM MIT KONDENSIERTEN ABBAUPRODUKTEN PFLANZLICHER UND TIERISCHER HERKUNFT**
COSMETIC AGENT WITH CONDENSED DECOMPOSITION PRODUCTS OF PLANT AND ANIMAL ORIGIN
PRODUIT COSMETIQUE AVEC PRODUITS DE DECOMPOSITION CONDENSES D'ORIGINE VEGETALE ET ANIMALE

(30) Priorität: 23.03.1995 DE 19510584; 05.02.1996 DE 19605032
(43) Veröffentlichungstag der Anmeldung: 07.01.1998
(73) Patentinhaber: Lancaster Group AG, 67059 Ludwigshafen (DE)
(72) Erfinder: GOLZ, Karin, MC-98000 Monaco (MC); ZASTROW, Leonhard, MC-98000 Monaco (MC); STANZL, Klaus, White Plains, NJ 10605 (US); KLÜGEL, Ulrich, D-12681 Berlin (DE); WESTPHAL, Günter, D-10319 Berlin (DE); SCHÖBEL, Leander, D-12557 Berlin (DE)
(74) Vertreter: Walter, Wolf-Jürgen
(86) Internationale Anmeldenummer: DE9600548
(87) Internationale Veröffentlichungsnummer: WO9629048

(56) Entgegenhaltungen:
- EP-A- 0 417 619
- DD-A- 273 276
- DE-A- 4 403 774

## Beschreibung

Die Erfindung betrifft kosmetische Grundstoffe mit Kondensaten von pflanzlichen und tierischen Abbauprodukten, die in kosmetischen Präparaten zur Körperpflege und Reinigung eingesetzt werden können und die neben der emulgierenden Wirkung wesentliche hautpflegende Eigenschaften haben.

Kosmetische Grundstoffe mit emulgierender und waschaktiver Wirkung sind in vielfältigen Kombinationen für die Verwendung in kosmetischen Mitteln zur Körperpflege und -reinigung bekannt geworden. Unter diesen haben insbesondere zwei Tensidklassen, die sich durch eine gute Hautverträglichkeit auszeichnen und aus natürlichen Rohstoffen zugänglich sind, zunehmend an Interesse gewonnen, nämlich die Eiweiß-Fettsäure-Kondensate und die Alkylpolyglycoside. Die Eiweiß-Fettsäure-Kondensate werden durch Umsetzung von Eiweiß-Partialhydrolysaten mit Fettsäuren, Fettsäurechloriden oder Fettsäureanhydriden in wäßriger Lösung unter Zusatz von Basen erhalten. Als Fettsäurekomponenten finden Derivate von pflanzlichen Fettsäuren (Kokosölfettsäure, ölsäure und andere) oder solche von synthetischen Fettsäuren mit identischen Kettenlängen Verwendung.

Die entsprechenden Eiweißhydrolysate werden durch alkalische, saure oder enzymatische Hydrolyse von natürlichen Proteinen oder proteinhaltigen Rohstoffen gewonnen. Ausgangsstoffe sind pflanzliche, tierische, mikrobielle und synthetische Proteine, beispielsweise Casein, Albumin, Knochenleim, Gelatine, Keratin, Lederabfälle, Kollagen, Seidenpeptide sowie Biomassen auf Paraffinbasis. Für den alkalischen Aufschluß eiweißhaltiger Stoffe wurden Alkali- oder Erdalkaliverbindungen, wie deren Hydroxide oder auch Ammoniak bei erhöhter Temperatur und gegebenenfalls erhöhtem Druck verwendet.

Die vorteilhaften Eigenschaften der Alkylpolyglycoside und der Eiweiß-Fettsäure-Kondensationsprodukte für kosmetische Anwendungen bestehen in ihrer Zugänglichkeit aus natürlichen Rohstoffen, deren Ethylenoxid-Freiheit, ihrer Eigenschaft zur Viskositätserhöhung von Gemischen, dem guten Schaumvermögen, dem haarkonditionierenden Eigenschaften, ihrer synergistischen Wirkung mit anderen Tensiden und nicht zuletzt ihrer biologischen Abbaubarkeit und guten dermatologischen Verträglichkeit. Bei diesen Produkten sind jedoch die ursprünglichen biologischen Strukturen im wesentlichen nicht mehr enthalten.

Der Erfindung liegt die Aufgabe zugrunde, Kosmetikgrundstoffe bzw. Kosmetika mit sehr guter Haut- und Schleimhautverträglichkeit sowie hautpflegenden Eigenschaften aus pflanzlichen und tierischen Ausgangsmaterialien bereitzustellen, die wesentliche Anteile an ursprünglichen biologischen Strukturen enthalten, jedoch soweit verändert sind, daß sie durch neutralen Geruch und helle Farbe kosmetisch annehmbar sind.

Erfindungsgemäß wird diese Aufgabe durch ein Kosmetikum gelöst, das ein ungereinigtes, kosmetisch wirksames Produkt eines direkten und milden (20 bis 55 °C; pH 7,5 - 8,5) Abbaus eines biologischen Ausgangsmaterials, ausgewählt aus der Gruppe, die aus Hefen, Hefefraktionen, Hülsenfrüchten, Hülsenfrüchtefraktionen, Pektinen, pektinhaltigen Massen, Algen, Algenfraktionen, Tiermilch, Tiermilchfraktionen und deren Gemischen besteht, im wäßrigen Medium und kondensiert mit einer unterstöchiometrischen Menge eines C₁₀-C₂₀-Fettsäurehalogenids oder eines C₁₀-C₂₀-Fettsäurehalogenidgemisches enthält, wobei das kondensierte Abbauprodukt biologische Strukturen des Ausgangsmaterials enthält, ausgewählt aus der Gruppe, die aus helicalen Naturstoffkomponenten, Enzymstrukturen, Vitaminstrukturen und deren Gemischen besteht.

Wenn das biologische Ausgangsmaterial eine Hefe oder Hefefraktion ist, so wird diese ausgewählt aus der Gruppe, die aus Bäckerhefe und Brauereihefe besteht. Es können auch Brauereiabfallhefen mit einem Feststoffgehalt von 10 bis 30 Gew.-% oder Hefebiomassefraktionen mit Hefezellresten, wie sie nach der Proteinextraktion in der hefeverarbeitenden Industrie als Nebenprodukte anfallen, eingesetzt werden.

Wenn das biologische Ausgangsmaterial eine Hülsenfrucht oder Hülsenfruchtfraktion ist, so wird diese ausgewählt aus der Gruppe, die aus Erbsen, Linsen, Sojabohnen und Ackerbohnen besteht.

Wenn das biologische Ausgangsmaterial ein Pektin oder eine pektinhaltige Masse ist, so sind dies insbesondere Citrusschalen und Apfeltrester sowie Traubentrester.

Wenn das biologische Ausgangsmaterial eine Alge oder eine Algenfraktion ist, so wird diese aus der Gruppe ausgewählt, die aus Grünalgen, Braunalgen und Rotalgen besteht, wobei diese Algen hohe Anteile an Chlorophyll, Alginaten, Proteinen und Mineralstoffen enthalten.

Wenn das biologische Ausgangsmaterial Tiermilch oder eine Tiermilchfraktion ist, so wird diese ausgewählt aus der Gruppe, die aus Stutenmilch, Kuhmilch, Schafmilch, Rentiermilch und Ziegenmilch besteht.

Ein besonders bevorzugtes Ausgangsmaterial ist Stutenmilch.

Biologische Ausgangsmaterialien, deren Bestandteile nicht direkt der chemischen Reaktion zugänglich sind, da sie in ihrer biologischen Zellstruktur vorliegen, werden durch Einwirkung von Ultraschall und/oder mechanischen Scherkräften homogenisiert und aufgeschlossen. Das hat gegenüber dem alkalischen Aufschluß von Zellstrukturen des Standes der Technik den Vorteil, daß weitgehend Enzym- und Vitaminaktivitäten erhalten bleiben. Die Ultraschallbehandlung erfolgt bei Temperaturen nicht über 55 °C und bei pH-Werten zwischen 6 und 8. Die Ultraschallbehandlung ist besonders geeignet bei biologischen Ausgangsmaterialien wie Hefen und Hülsenfrüchten.

Das biologische Ausgangsmaterial, das gegebenenfalls durch Ultraschall oder mechanische Scherkräfte vorbehandelt wurde, wird auf einen Feststoffgehalt von 15 bis 30 Gew.-% und einen pH-Wert von 7,5 bis 8,5 eingestellt. Danach erfolgt der Umsatz der Suspensionen mit einer unterstöchiometrischen Menge an Säurehalogeniden pflanzlicher Fettsäuren oder synthetischer Fettsäuren mit Kettenlängen von 10 bis 20 Kohlenstoffatomen, vorzugsweise 12 bis 18 Kohlenstoffatomen bei Temperaturen von 20 bis 55 °C (Kondensation). Es können auch Fettsäuren mit unterschiedlichen Kettenlängen aus dem oben genannten Bereich eingesetzt werden.

Die Einstellung des pH-Wertes im alkalischen Bereich erfolgt vorzugsweise mit Alkalimetallhydroxidlösungen oder Alkalicarbonaten. Die Zugabe von entsprechenden Erdalkaliverbindungen ist ebenfalls möglich, jedoch hat dies den Nachteil, daß die entsprechenden Salze als unlösliche Bestandteile im Endprodukt verbleiben.

Von den Fettsäurehalogeniden sind die Chloride besonders bevorzugt. Als Fettsäuren sind solche mit 12 bis 18 Kohlenstoffatomen bevorzugt. Eine besonders bevorzugte Fettsäure ist Palmitinsäure. Nach der Umsetzung des biologischen Ausgangsmaterials mit dem Fettsäurehalogenid oder Fettsäurehalogenidgemisch können die so erhaltenen Produkte zur Verbesserung ihrer Eigenschaften oder zur Anpassung an spezifische Verwendungszwecke, beispielsweise zur Steigerung der Gelierfähigkeit, durch Einführung geeigneter Substituenten mit Hilfe von Veresterungen oder Veretherungen modifiziert werden. Als besonders geeignet erwiesen sich Carboxymethylierungen mit Mono- oder Dichloressigsäure oder Succinylierungen mit Bernsteinsäureanhydrid.

Die erfindungsgemäßen biologisch abgebauten und kondesierten Produkte sind in erster Linie bedeutsam in Kosmetika wegen des weitgehenden Erhalts biologischer Strukturen, wie Helices, Enzym-und Vitaminstrukturen, und sie unterscheiden sich damit deutlich von herkömmlichen Produkten. Die Produkte weisen auch underivatisierte Bestandteile des biologischen Ausgangsmaterials bzw. deren Spaltprodukte auf, beispielsweise Peptide, Aminosäuren, Kohlenhydrate und Fette, da die als Reaktionspartner verwendeten Fettsäurehalogenide in unterstöchiometrischen Mengen eingesetzt wurden. Diese Spaltprodukte der biologischen Ausgangsmaterialien besitzen vorteilhafte Wirkungen auf die menschliche Haut.

Als helicale Naturstoffkomponenten im Zusammenhang mit der vorliegenden Erfindung werden Proteine und Polysaccharide verstanden, die Einzelhelixbereiche oder Doppelhelixstrukturen ausbilden.

Weiterhin haben die biologischen Ausgangsprodukte eine emulgierende Wirkung, und können als im wesentlichen nichtionische Tenside mit anionischen Anteilen mit dieser Nebenwirkung in Cremes, Waschlotionen, Haarwaschmitteln, Haarkonditionierungsmitteln, Masken oder Gelen vorteilhaft eingesetzt werden. Weiterhin können sie als Wirkstoffstabilisatoren oder als viskositätssteigernde Komponente in Cremes oder Lotionen verwendet werden und zeigen insgesamt eine ausgezeichnete Haut- und Schleimhautverträglichkeit sowie pflegende Eigenschaften.

Wegen der Unterschiede der Bestandteile in den biologischen Ausgangsmaterialien im Hinblick auf ihre chemische Beschaffenheit und ihre Molekülgröße entsteht bei dem erfindungsgemäßen milden Abbau mit nachfolgender Kondensation ein Gemisch verschiedener chemischer Verbindungen mit breiter Molekulargewichtsverteilung. Dadurch können Instabilitäten von kosmetischen Präparaten auch bei variierenden Mengenverhältnissen weitestgehend vermieden werden.

Besonders vorteilhaft ist die Tatsache, daß die wäßrigen Lösungen der erfindungsgemäßen kondensierten Abbauprodukte aus biologischen Ausgangsmaterialien geringere Oberflächenspannungen als solche Tenside haben, die auf Basis nachwachsender Rohstoffe wie Eiweiß-Fettsäure-Kondensate oder Alkylpolyglycoside hergestellt werden.

Die Erfindung betrifft auch ein Verfahren zur Herstellung von kosmetisch wirksamen, kondensierten Abbauprodukten pflanzlicher und tierischer Herkunft, das dadurch gekennzeichnet ist, daß man ein biologisches Ausgangsmaterial, ausgewählt aus der Gruppe, die aus Hefen, Hefefraktionen, Hülsenfrüchten, Hülsenfrüchtefraktionen, Pektinen, pektinhaltigen Massen, Algen, Algenfraktionen, Tiermilch und Tiermilchfraktionen sowie deren Gemischen besteht, im wäßrigen und schwach basischen Medium im Bereich von pH 7,5 bis 8,5 im Temperaturbereich von 20 bis 55 °C abbaut und mit einer unterstöchiometrischen Menge eines Halogenids einer C₁₀-C₂₀-Fettsäure oder eines Fettsäurehalogenidgemisches solcher Fettsäuren kondensiert, danach den pH-Wert auf Werte im Bereich von 5 bis 7 einstellt und das erhaltene Produkt, das biologische Strukturen des Ausgangsproduktes enthält, ausgewählt aus der Gruppe, die aus helicalen Naturstoffkomponenten, Enzymstrukturen und Vitaminstrukturen und deren Gemischen besteht, ohne weitere Trennung homogenisiert und gegebenenfalls das Homogenisat mit weiteren kosmetischen Stoffen der Gruppe Trägerstoffe, Hilfsstoffe, Wirkstoffe vermischt und gegebenenfalls zu einer kosmetischen Zubereitung verarbeitet, wobei die Temperatur 55 °C nicht überschreitet und der pH-Wert des finalen Produktes zwischen pH 5 und 7 liegt.

Besonders vorteilhaft erfolgt die Einstellung des pH-Wertes im schwach basischen Bereich von pH 7,5 bis 8,5 mit einem Alkalihydroxid oder Alkalicarbonat.

Besonders vorteilhaft erfolgt die Einstellung des pH-Wertes im schwach sauren Bereich pH 5 bis 7 mit einer Säure, die aus der Gruppe ausgewählt ist, die aus Salzsäure, Weinsäure, Äpfelsäure und Citronensäure besteht.

Vorteilhaft werden biologische Strukturen, die nicht direkt der chemischen Reaktion zugänglich sind, durch Einwirkung von Ultraschall und/oder mechanische Scherkräfte teilweise aufgeschlossen, wobei die Temperatur beim Aufschluß 55 °C nicht übersteigt und der pH im Bereich von 5 bis 8 liegt.

Ein besonders vorteilhaftes Aufschlußprodukt rührt aus einem Ultraschallaufschluß mit einer Ultraschall-Durchflußzelle gemäß der DE 42 41 154 her, bei der die Sonotrode zu ½ bis ²/₃ ihrer Länge in die Durchflußzelle hineinragt, der Winkel der Sonotrode im Beschallungsgefäß im Bereich von 80,5 bis 88,5° liegt, das Verhältnis der Eintauchlänge der Synotrode (in mm) zum Beschallungsvolumen (in ml) auf einen Wert im Bereich von 1:1,1 bis 1:20 eingestellt ist und das Verhältnis von Eintauchlänge der Synotrode (in mm) zu dem Feststoffanteil des zu beschallenden Mediums (in Masse-%) im Bereich von 1:0,02 bis 1:2,2 liegt.

Erfindungsgemäß kann das biologische Abbauprodukt nach der Umsetzung mit Fettsäurehalogenid weiterhin verestert oder verethert werden, um dadurch spezielle Anwendungszwecke zu erschließen, beispielsweise eine Verbesserung der Gelierfähigkeit herbeizuführen, oder andere Eigenschaften zu beeinflussen. Vorteilhaft ist die Carboxymethylierung mit Mono- oder Dichloressigsäure oder die Succinylierung mit Bernsteinsäureanhydrid.

Zur Einstellung des pH-Wertes im schwach sauren Bereich können z. B. die obigen Säuren eingesetzt werden. Gegenüber Salzsäure besitzen jedoch Hydroxycarbonsäuren wie Weinsäure, Äpfelsäure und besonders bevorzugt Citronensäure den Vorteil, daß sie im Verlaufe der Neutralisation bei den Produkten eine Aufhellung bewirken. Zusätzlich kann jedoch auch Wasserstoffperoxid zugesetzt werden, um eine weitere Verbesserung von Farbe und Geruch zu bewirken. Dadurch können Produkte mit milchig-weißer bis milchig-hellgelber bzw. hellgrüner bzw. hellbrauner Farbe je nach dem Ausgangsprodukt Tiermilch bzw. Hefe bzw. Grünalgen oder grüne Erbsen bzw. Braunalgen hergestellt werden, die einen neutralen oder angenehmen Geruch haben.

Das nach dem erfindungsgemäßen Verfahren abgebaute und kondensierte biologische Ausgangsmaterial kann ohne weitere Reinigung in den Kosmetika als kosmetischer Grundstoff mit pflegenden Eigenschaften, als Tensid bzw. zusätzlicher Tensidbestandteil und als Regulator der Oberflächenspannung verwendet werden. Es stellt aber auch bereits selbst - bei Zusatz nur weniger üblicher Kosmetikhilfsstoffe - ein Kosmetikum dar infolge seines hohen Anteiles an Wirkstoffen.

Von den bisher bekannten Produkten unterscheidet sich der erfindungsgemäße Kosmetikgrundstoff deutlich durch hohe Anteile biologischer Strukturen, die durch die Einstellung des Feststoffgehaltes des biologisch abgebauten Produktes im Endprodukt in breitem Umfang gesteuert werden können. So liegt dieser Feststoffgehalt bei eingesetzter Tiermilch im Bereich von 35 bis 50 Gew.-%, bezogen auf die Gesamtmasse, um eine gute Konsistenz des Endproduktes zu erhalten. Bei Leguminosen liegt der Feststoffgehalt etwa im Bereich von 15 bis 25 Gew.-%, um eine gute Konsistenz des Endproduktes zu haben.

Der Anteil der Helixstrukturen im abgebauten biologischen Material kann über die sog. Helix-coil-Übergangsanalyse gemessen werden (Kogan et al., Biopolymers Vol. 27, 1055-63; Williams et al., Carb. res. 219, 203-213), bei der die Tatsache genutzt wird, daß geordnete Strukturen, wie Helices, Komplexe mit z.B. Farbstoffen bilden. Gegenüber einer Vergleichssubstanz, wie Stärke, können Absorptionsmaxima bei entsprechenden NaOH-Konzentrationen gemessen werden. Damit ist es möglich, gezielt Quantitäten von Helices über die Menge an eingesetztem Fettsäurehalogenid einzustellen.

Der Begriff "unterstöchiometrische Menge" im Zusammenhang mit dieser Erfindung bedeutet, daß, bezogen auf das Gewicht, 20 bis 90 % Fettsäurehalogenid gegenüber 100 % biologisches Ausgangsmaterial eingesetzt werden. Für Tiermilch, insbesondere Stutenmilch, liegt ein vorteilhafter Bereich der unterstöchiometrischen Menge bei z.B. 50 bis 70 %.

Die Einstellung eines unterstöchiometrischen Verhältnisses kann über die Bestimmung der funktionellen Gruppen des Ausgangsmaterials erfolgen, wobei funktionelle Gruppen vorrangig primäre und sekundäre Hydroxy- und Aminogruppen sind. Diese Einstellung des Verhältnisses erfolgt somit über die OH-Zahl und Amin-Zahl (in gleicher Weise wie bei der Polyurethanherstellung), indem entsprechend der festgestellten OH- bzw. Amin-Zahl einer bestimmten Menge Ausgangsmaterial (100 %) eine geringere Menge Fettsäurehalogenid (20 bis 90 %) hinzugesetzt wird. Je nachdem welche Feststoffgehalte im Endprodukt erwünscht sind - und damit bestimmte Viskositäten - kann über die Höhe des Unterschusses an Fettsäurehalogenid(gemisch) dieser Feststoffgehalt gesteuert werden und damit der Anteil an den biologischen Strukturen.

Das Kosmetikum kann vorteilhaft in Form einer Creme, einer Körperwaschlotion, eines Haarwaschmittel, eines Haarkonditionierungsmittels, einer Maske oder eines Gels vorliegen.

Es kann aber auch direkt als Produkt der Kondensation oder der nachfolgenden Veretherung oder Veresterung vorliegen.

Die Erfindung soll nachstehend durch Beispiele näher erläutert werden. Diese Beispiele schränken die Erfindung jedoch nicht ein.

### Beispiel 1

333 g Bäckerhefe (30 Gew.-% Trockenmasse) wurde mit 166 ml Wasser suspendiert und eine Stunde mit Ultraschall behandelt (Gerät USD 30 der Emich Ultraschall GmbH, Generatorleistung 400 W, Amplitude 50 *µ*m). Durch Kühlung der Beschallungszelle wurde die Temperatur unter 30 °C gehalten. Nach der Behandlung lag eine gelbrosa, viskose Suspension mit einem pH-Wert von 5,5 vor. Der pH-Wert wurde mit Natriumhydroxid auf 8,0 eingestellt und unter Rühren wurden 60 g Palmitinsäurechlorid langsam hinzugegeben. Die Temperatur wurde auf 50 °C und der pH-Wert durch Zugabe von Natriumhydroxid auf 8,0 gehalten. Nach 90 Minuten wurden 10 ml Wasserstoffperoxid (30 %) zugesetzt und das Gemisch auf Raumtemperatur abgekühlt.

Die aufgeschäumte Masse wurde mit Citronensäure auf einen pH-Wert von 5,5 eingestellt und homogenisiert.

Das Produkt hatte eine hellgelbe bis hellgraue Farbe, einen angenehmen rohstoffidentischen Geruch und einen Feststoffgehalt von 34 Gew.-%.

### Beispiel 2

150 g einer industriellen Hefeproteinfraktion (DHW Hamburg) wurden in 850 ml Wasser gelöst, auf einen pH-Wert von 8,0 eingestellt und unter Rühren bei 55 °C mit 75 g Stearinsäurechlorid versetzt. Es wurde 60 Minuten unter Einhaltung von Temperatur und pH-Wert (NaOH-Zugabe) gerührt. 15 ml Wasserstoffperoxid wurden beim Abkühlen zugesetzt und nach 15 Minuten mit Äpfelsäure ein pH-Wert von 6,0 eingestellt.

Das Produkt hatte eine hellbraune Farbe und einen Feststoffgehalt von 21 Gew.-%.

### Beispiel 3

300 g trockene grüne Schälerbsen wurden in 1700 ml Wasser 24 Stunden bei Raumtemperatur gequollen und danach mechanisch (Ultra-Turrax) homogenisiert. Die Masse wurde mit Natriumhydroxid auf einen pH-Wert von 8,3 eingestellt und 60 Minuten bei 55 °C gerührt. Es wurden 144 g Ölsäurechlorid langsam zugesetzt und der pH-Wert durch Zugabe von Kaliumhydroxid konstant gehalten. Nach 90 Minuten Rühren bei 45 °C wurden 20 ml Wasserstoffperoxid zugesetzt, die Masse auf Raumtemperatur abgekühlt, mit Citronensäure auf einen pH-Wert von 6,0 eingestellt und homogenisiert.

Die cremige Masse war hellgrün und hatte einen Feststoffgehalt von 22 Gew.-%.

### Beispiel 4

500 g trockene Bohnen wurden in 2000 ml Wasser 24 Stunden bei Raumtemperatur gequollen und danach mechanisch homogenisiert. Der pH-Wert der Masse wurde durch Zugabe von Natriumhydroxid auf 8,0 eingestellt, 90 Minuten bei 50 °C gerührt und bei 10000 U/min zentrifugiert. Der überstand, der 1600 g einer gelblichen, leicht trüben Flüssigkeit mit einem Feststoffgehalt von 18 Gew.-% darstellte, wurde bei 50 °C unter Einhaltung eines pH-Wertbereiches von 7,5 bis 8,0 (unter Zusatz von Natriumhydroxid) mit 140 g Palmitinsäure versetzt.

Nach einer Reaktionszeit von 60 Minuten wurden 30 g Monochloressigsäure-Natriumsalz zugesetzt, und es wurde weitere 30 Minuten gerührt. Beim Abkühlen wurden 20 ml Wasserstoffperoxid (30 %) zugesetzt, und nach dem Abkühlen auf 25 °C wurde mit Citronensäure ein pH-Wert von 5,5 eingestellt.

Das Produkt bestand aus einer hochviskosen, weißen Paste mit einem Feststoffgehalt von 27 Gew.-%.

### Beispiel 5

Es wurde wie im Beispiel 3 verfahren, jedoch wurden statt Erbsen Sojabohnen und statt 144 g ölsäurechlorid wurden 120 g Palmitinsäurechlorid verwendet. Nach der Reaktion mit dem Säurechlorid wurden unter Einhaltung eines pH-Wertes von 8,5 bei 45 °C 1,5 g Bernsteinsäureanhydrid unter Rühren zugesetzt. Danach wurde weitere 90 Minuten gerührt. Diese Masse wurde abgekühlt und mit Citronensäure auf einen pH-Wert von 6,0 eingestellt. Es entstand eine cremige, hellgelbe Masse mit einem Feststoffgehalt von 19 Gew.-%.

### Beispiel 6

16 g Apfelpektin (Veresterungsgrad 50 %) wurden in 184 ml Wasser gelöst, durch Zugabe von Natriumhydroxid ein pH-Wert von 8,0 eingestellt, und unter Rühren wurden bei 55 °C 10 g Laurinsäurechlorid zugesetzt. Die Mischung wurde unter Einhaltung der Reaktionsbedingungen 90 Minuten gerührt. Danach wurde abgekühlt, 2 ml Wasserstoffperoxid (30 %) zugesetzt und nach 15 Minuten mit Citronensäure ein pH-Wert von 6,0 eingestellt.

Das Produkt war eine hell ockerfarbene cremige Masse mit einem Feststoffgehalt von 15 Gew.-%.

### Beispiel 7

300 g ungespritzte Zitrusfruchtschalen wurden mechanisch homogenisiert, die Masse mit Natriumhydroxid auf einen pH-Wert von 8,5 eingestellt und bei 50 °C 30 Minuten gerührt. Es wurden 36 g Palmitinsäurechlorid unter Einhaltung der Reaktionsbedingungen zugesetzt und 60 Minuten die Gesamtmasse gerührt. Nach dem Abkühlen wurde der pH-Wert mit Citronensäure auf 5,5 eingestellt. Das Produkt war eine hellgelbe cremige Masse mit einem Feststoffgehalt von 27 Gew.-%.

### Beispiel 8

Ein Grünalgenextrakt wurde im Wasser gelöst, die Lösung mit Natriumhydroxid auf einen pH-Wert von 8,0 bis 8,5 eingestellt und unter Rühren auf 45 °C erwärmt. Danach wurden 6-10 Gew-% Palmitinsäurechlorid unter weiterem Rühren bei 45 ° C und einem pH-Wert von 8 - 8,5 zugesetzt und die Masse zwei Stunden gerührt. Mit Citronensäure wurde ein pH-Wert von 5,0 bis 6,5 eingestellt und die Masse homogenisiert. Das Produkt war eine hellgelbe Masse, die je nach Menge des Grünalgenextraktes und Menge der Palmitinsäure einen Feststoffgehalt von 20 bis 50 % hatte.

### Beispiel 9

Stutenmilchtrockenpulver wurde in Wasser suspendiert und mit Natriumhydroxid ein pH-Wert von 7,5 bis 8,3 eingestellt. Das Gemisch wurde auf 50 °C erwärmt und unter Rühren wurden in 30 Minuten Kokosfettsäurechlorid zugesetzt. Der pH-Wert wurde durch Zugabe von Natriumhydroxid dabei zwischen 7,5 und 8,5 gehalten, und es wurden weitere 30 Minuten gerührt. Beim Abkühlen wurde mit Äpfelsäure oder mit Citronensäure ein pH-Wert von 5,0 bis 6,0 eingestellt. Das Produkt war weiß und hatte einen Feststoffgehalt von 4,5 bis 30 Gew.-% entsprechend der eingesetzten Menge an Stutenmilch und Kokosfettsäurechlorid.

### Beispiel 10

300 g Molkenprotein wurde in Wasser gelöst, die Lösung mit Natriumcarbonatlösung auf ein pH-Wert von 7,0 bis 8,0 eingestellt und unter Rühren auf 45 °C erwärmt. Es wurde langsam 100 g Stearinsäurechlorid und 80 g Laurinsäurechlorid zugesetzt, und der pH-Wert wurde durch Zugabe von Natriumcarbonatlösung auf 7,0 bis 8,0 gehalten. Die Masse wurde nach zwei Stunden Reaktionszeit auf Raumtemperatur abgekühlt und der pH-Wert mit Citronensäure auf 7,0 eingestellt. Das weiße Produkt hatte einen Feststoffgehalt von 30 Gew.-%.

### Beispiel 11

Eine Kombination aus 0,2 Gewichtsteilen Molkenprotein und 0,8 Gewichtsteilen Stutenmilchpulver wurde in Wasser gelöst und der pH-Wert auf 7,5 eingestellt. Anschließend wurde unter Rühren auf etwa 45 °C erwärmt, und es wurden 40 % Palmitinsäurechlorid, bezogen auf das Gewicht des Einsatzproduktes, der Mischung zugesetzt und zwei Stunden gerührt. Nach Abkühlung wurde der pH-Wert auf 6,3 eingestellt. Man erhielt eine nahezu weiße Suspension, die nach Zugabe von Citronensäure bis zum pH 6,0 vollständig weiß wurde und einen Feststoffgehalt von 25 Gew.-% aufwies.

### Beispiel 12

Es wurde wie in Beispiel 11 gearbeitet, jedoch eine Kombination von 0,7 Gewichtsteilen Molkenprotein und 0,3 Gewichtsteilen Stutenmilchpulver eingesetzt. Man erhielt eine weiße Masse mit einem Feststoffgehalt von 22 Gew.-%.

### Beispiel 13

Es wurde wie im Beispiel 11 gearbeitet, jedoch als biologisches Ausgangsmaterial ein Gemisch von 1 bis 5 Gewichtsteilen Grünalgen, 2 bis 7 Gewichtsteilen Braunalgen und 3 bis 10 Gewichtsteilen Rotalgen eingesetzt. Man erhielt eine hellbraune Masse mit einem Feststoffgehalt von 28 Gew.-%.

### Beispiel 14

Es wurde ein Gemisch aus 0,5 Gewichtsteilen Molkenprotein und 99,5 Gewichtsteilen Stutenmilchtrockenpulver eingesetzt, in Wasser gelöst und mit Natriumhydroxid auf einen pH-Wert von 7,8 eingestellt. Das Gemisch wurde unter Rühren auf 45 °C erwärmt. Nach Zugabe von Kokosfettsäurechlorid wurde der pH-Wert bei 7,5 gehalten. Nach einer Reaktionszeit von 2,2 Stunden unter Rühren bei 45 °C wurde die Masse abgekühlt. Die Abkühlung erfolgte sehr langsam. Der pH wurde anschließend mit Äpfelsäure auf 6,0 eingestellt. Das Produkt war weiß und hatte einen Feststoffgehalt von 24 Gew.-%.

### Beispiel 15

Es wurde wie im Beispiel 14 gearbeitet, wobei das Ausgangsmaterial eine Kombination von 67,6 Gew.-% Molkenprotein und 32,3 Gew.-% Stutenmilchpulver war. Als Fettsäurehalogenid wurde ein Gemisch von Stearinsäurechlorid und Laurinsäurechlorid 1:1 eingesetzt. Man erhielt als Produkt eine weiße Masse mit einem Feststoffgehalt von 30 Gew.-%.

### Beispiel 16

Es wurde eine Kombination von Rotalgen und Braunalgen im Verhältnis 7,5 : 92,5 eingesetzt und wie im Beispiel 11 gearbeitet, wobei als Fettsäurehalogenid Laurinsäurechlorid verwendet wurde. Der pH-Wert im basischen Medium wurde auf 8,5 eingestellt.

Man erhielt ein blaß ockerfarbenes Produkt mit einem Feststoffgehalt von 35 Gew.-%.

### Beispiel 17

### Haarshampoo und Haarkonditionierungsmittel "2 in 1" (Bezeichnungen als CTFA-Namen)

| Phase A | |
|---|---|
| Cocamidopropyl Betaine | 10 % |
| Sodium Lauryl Sulfoacetate | 25 % |
| Destilliertes Wasser | q.s. |
| Parfümöl | |
| Konservierungsmittel | |

| Phase B | |
|---|---|
| Produkt nach Beispiel 11 | 12,5 % |

Die Herstellung der Phase A erfolgte, indem Wasser vorgelegt wurde und unter Rühren die Zugabe von Cocamidopropyl Betaine, Sodium Lauryl Sulfoacetate, Parfümöl und Konservierungsmittel durchgeführt wurde. Anschließend wurde das Gemisch gut miteinander verrührt. Danach wurde die Phase B auf 38 °C erwärmt und der Phase A unter Rühren zugegeben. Anschließend erfolgte eine Homogenisierung des Gemisches.

### Beispiel 18

### Haarshampoo und Haarkonditionierungsmittel "2 in 1"

Es wurde wie im Beispiel 17 gearbeitet, jedoch die folgende Phase B verwendet.

| Phase B | |
|---|---|
| Produkt nach Beispiel 1 | 2,5 % |
| Produkt nach Beispiel 11 | 10,0 % |

### Beispiel 19

### Körpercreme

| Phase A | |
|---|---|
| Glyceryl Stearate/Ceteraceth-22-Ceteareth 12-Cetearyl Alcohol Cetyl Palmitate | 3,0 % |
| Cetearyl Alcohol | 2,0 % |
| Jojobaöl | 1,0 % |

| Phase B | |
|---|---|
| Destilliertes Wasser | q.s. |
| Propylenglycol | 2,0 % |
| Glycerin | 1,0 % |

| Phase C | |
|---|---|
| Produkt nach Beispiel 9 | 1,5 % |
| Produkt nach Beispiel 10 | 2,0 % |
| Produkt nach Beispiel 14 | 2,5 % |
| Konservierungsmittel | 0,3 % |
| Parfümöl | |

Die Herstellung der Phasen A und B erfolgte separat unter Rühren bei etwa 60 ± 5 °C. Danach wurden beide miteinander vermischt und homogenisiert. Die Phase C wurde bei einer Temperatur von gleich oder kleiner als 40 °C in dem Gemisch der Phasen A und B verteilt und anschließend homogenisiert. In gleicher Weise wie im Beispiel 19 wurde eine Körperlotion und eine kosmetische Maske hergestellt.

### Beispiel 20

### Gel

| | |
|---|---|
| Acrylates C₁₀-C₃₀-Alkyl Acrylate Crosspolymer | 1,0 % |
| TEA | 1,0 % |
| Octyl Stearate | 2,5 % |
| Produkt von Beispiel 8 | 5,0 % |
| Konservierungsmittel | 0,3 % |
| Parfümöl (PO) nach Bedarf | |
| Produkt von Beispiel 16 | 2,5 % |
| Dest. Wasser | q.s. |

Die Herstellung erfolgte, indem das Gel bei Raumtemperatur in Wasser vordispergiert und danach neutralisiert wurde. Dann erfolgte die Zugabe von öl und den Produkten der Beispiele 8 und 16. Das Ganze wurde gut homogenisiert und zum Abschluß mit PO und Konservierungsstoffen versetzt.

## Patentansprüche

1. Kosmetikum aus kondensierten Abbauprodukten pflanzlicher und tierischer Herkunft, **dadurch gekennzeichnet, daß** es ein Produkt eines direkten und milden (20 - 55 °C; pH 7,5 - 8,5) Abbaus eines biologischen Ausgangsmaterials im wäßrigen Medium, einer nachfolgenden Kondensation mit einer unterstöchiometrischen Menge eines C₁₀-C₂₀-Fettsäurehalogenids oder eines C₁₀-C₂₀-Fettsäurehalogenidgemisches und gegebenenfalls einer nachfolgenden Veretherung oder Veresterung des Kondensationsproduktes ist,
wobei das Ausgangsmaterial aus der Gruppe ausgewählt ist, die aus Hefen, Hefefraktionen, Hülsenfrüchten, Hülsenfrüchtefraktionen, Pektinen, pektinhaltigen Massen, Algen, Algenfraktionen, Tiermilch, Tiermilchfraktionen und deren Gemischen besteht,
und wobei das kondensierte Abbauprodukt biologische Strukturen des Ausgangsmaterials enthält, ausgewählt aus der Gruppe, die aus helicalen Naturstoffkomponenten, Enzymstrukturen und Vitaminstrukturen besteht,
und wobei das Kondensationsprodukt gegebenenfalls in einer kosmetischen Zubereitung mit weiteren kosmetischen Hilfs-, Träger oder Wirkstoffen vorliegt.

2. Kosmetikum nach Anspruch 1, **dadurch gekennzeichnet, daß** das biologische Ausgangsmaterial eine Hefe oder Hefefraktion ist, ausgewählt aus der Gruppe, die aus Bäckerhefe und aus Brauereihefe besteht.

3. Kosmetikum nach Anspruch 1, **dadurch gekennzeichnet, daß** das biologische Ausgangsmaterial eine Hülsenfrucht oder Hülsenfruchtfraktion ist, ausgewählt aus der Gruppe, die aus Erbsen, Linsen, Sojabohnen und Ackerbohnen besteht.

4. Kosmetikum nach Anspruch 1, **dadurch gekennzeichnet, daß** das biologische Ausgangsmaterial ein Pektin oder eine pektinhaltige Masse ist, ausgewählt aus der Gruppe, die aus Zitrusschalen, Apfeltrester und Traubentrester besteht.

5. Kosmetikum nach Anspruch 1, **dadurch gekennzeichnet, daß** das biologische Ausgangsmaterial eine Alge ist, ausgewählt aus der Gruppe, die aus Grünalgen, Braunalgen, Rotalgen und deren Gemischen besteht mit hohen Anteilen an Chlorophyll, Alginaten, Proteinen und Mineralstoffen.

6. Kosmetikum nach Anspruch 1, **dadurch gekennzeichnet, daß** das biologische Ausgangsmaterial Tiermilch ist, ausgewählt aus der Gruppe, die aus Stutenmilch, Kuhmilch, Schafmilch, Rentiermilch, Ziegenmilch, Molkenprotein und Milchfraktionen besteht, und daß es bevorzugt Stutenmilch ist.

7. Kosmetikum nach einem der vorangegangenen Ansprüche, **dadurch gekennzeichnet, daß** es das Produkt eines direkten und milden Abbaus eines biologischen Ausgangsmaterials mit vorheriger mechanischer Scherbehandlung oder Ultraschallbehandlung des Ausgangsmaterials und der nachfolgenden Kondensation mit unterstöchiometrischen Mengen eines Fettsäurehalogenids ist.

8. Kosmetikum nach Anspruch 1, **dadurch gekennzeichnet, daß** das Kondensationsprodukt einen pH-Wert von 5 bis 7 hat.

9. Kosmetikum nach einem der vorangegangenen Ansprüche, **dadurch gekennzeichnet, daß** die kosmetische Zubereitung eine Creme, eine Körperwaschlotion, ein Haarwaschmittel, ein Haarkonditionierungsmittel, eine Maske oder ein Gel ist.

10. Kosmetikum nach einem der vorangegangenen Ansprüche, **dadurch gekennzeichnet, daß** es das Produkt eines direkten und milden Abbaus eines biologischen Ausgangsmaterials mit nachfolgender Kondensation mit unterstöchiometrischen Mengen eines Fettsäurehalogenids und einer nachfolgenden Carboxymethylierung oder Succinylierung ist.

11. Verfahren zur Herstellung eines Kosmetikums aus kondensierten Abbauprodukten pflanzlicher und tierischer Herkunft **dadurch gekennzeichnet, daß** man ein biologisches Ausgangsmaterial, ausgewählt aus der Gruppe, die aus Hefen, Hefefraktionen, Hülsenfrüchten, Hülsenfrüchtefraktionen, Pektinen, pektinhaltigen Massen, Algen, Algenfraktionen, Tiermilch, Tiermilchfraktionen und deren Gemischen besteht, in einem schwach basischem Medium im Bereich von pH von 7,5 bis 8,5 im Temperaturbereich von 20 bis 55 °C, gegebenenfalls nach vorheriger mechanischer Scherbehandlung oder Ultraschallbehandlung, abbaut und mit einer unterstöchiometrischen Menge eines Halogenids einer C₁₀-C₂₀-Fettsäure oder eines Fettsäurehalogenidgemisches einer solchen Fettsäure kondensiert, danach den pH-Wert auf Werte im Bereich von 5 bis 7 einstellt und das erhaltene Produkt ohne weitere Trennung homogenisiert, und gegebenenfalls das Homogenisat mit weiteren kosmetischen Stoffen der Gruppe Trägerstoffe, Hilfsstoffe, Wirkstoffe vermischt und zu einer kosmetischen Zubereitung verarbeitet, wobei die Temperatur für das biologische Abbauprodukt von 55 °C nicht überschritten wird und der pH-Wert des finalen Produktes zwischen pH 5 und 7 liegt.

12. Verfahren nach Anspruch 11, **dadurch gekennzeichnet, daß** die Einstellung des pH-Wertes im schwach basischen Bereich mit einem Alkalihydroxid oder Alkalicarbonat erfolgt.

13. Verfahren nach Anspruch 11, **dadurch gekennzeichnet, daß** die Einstellung des pH-Wertes im schwach sauren Bereich mit einer Säure erfolgt, die aus der Gruppe ausgewählt ist, die aus Salzsäure, Weinsäure, Äpfelsäure und Citronensäure besteht.

14. Verfahren nach Anspruch 11, **dadurch gekennzeichnet, daß** das biologische Ausgangsmaterial durch Einwirkung von Ultraschall oder von mechanischen Scherkräften oder von beiden homogenisiert und teilweise aufgeschlossen wird, wobei die Temperatur beim Aufschluß 55 °C nicht übersteigt und der pH-Wert im Bereich von 5 bis 8 liegt.

15. Verfahren nach einem der vorangegangenen Ansprüche, **dadurch gekennzeichnet, daß** das biologische Abbauprodukt nach der Umsetzung mit dem Fettsäurehalogenid verestert oder verethert wird.

16. Verfahren nach Anspruch 15, **dadurch gekennzeichnet, daß** das biologische Abbauprodukt mit Mono- oder Dichloressigsäure carboxymethyliert oder mit Bernsteinsäureanhydrid succinyliert wird.

17. Verfahren nach einem der vorangegangenen Ansprüche, **dadurch gekennzeichnet, daß** dem biologischen Abbauprodukt zur weiteren Entfärbung oder Geruchsbindung eine Menge Wasserstoffperoxid zugesetzt wird, die im Höchstfall quantitativ umzusetzen ist.

18. Verwendung eines Kosmetikums aus kondensierten Abbauprodukten pflanzlicher und tierischer Herkunft nach Anspruch 1 als Kosmetikgrundstoff.

19. Verwendung eines Kosmetikums aus kondensierten Abbauprodukten pflanzlicher und tierischer Herkunft nach Anspruch 1 zur Herstellung von kosmetischen Zubereitungen zusammen mit weiteren kosmetischen Hilf-, Träger und/oder Wirkstoffen.

## Claims

1. A cosmetic made of condensed plant and animal decomposition products, **characterized in that** it is a product of a direct and mild (20 - 55°C; pH 7.5 - 8.5) decomposition of a biological starting material in an aqueous solution, subsequent condensation with a sub-stoichiometric quantity of a C₁₀-C₂₀ fatty acid halide or a C₁₀-C₂₀ fatty acid halide mixture, and, if applicable, subsequent etherisation or esterisation of the condensation product,
in which situation the starting material is selected from the group which consists of the yeasts, yeast fractions, pulses, pulse fractions, pectins, pectin-containing masses, algae, algae fractions, animal milk, animal milk fractions, and their mixtures,
and in which situation the condensed decomposition product contains the biological structures of the starting material, selected from the group which consists of the helical natural substance components, enzyme structures, and vitamin structures,
and in which situation the condensation product occurs, if applicable, in a cosmetic preparation with additional ancillary, carrier, or active substances.

2. A cosmetic according to Claim 1, **characterized in that** the biological starting material is a yeast or yeast fraction, selected from the group which consists of baker's yeast and brewer's yeast.

3. A cosmetic according to Claim 1, **characterized in that** the biological starting material is a pulse or pulse fraction, elected from the group which consists of peas, lentils, soya beans and broad beans.

4. A cosmetic according to Claim 1, **characterized in that** the biological starting material is a pectin or pectin-containing mass, selected from the group which consists of citrus peel, pomace, and grape daff.

5. A cosmetic according to Claim 1, **characterized in that** the biological starting material is an algae, selected from the group which consists of green algae, brown algae, red algae, an their mixtures, with high proportions of chlorophyll, alginates, proteins and mineral substances.

6. A cosmetic according to Claim 1, **characterized in that** the biological starting material is animal milk, selected from the group which consists of mare's milk, cow's milk, sheep's milk, reindeer milk, goat's milk, whey protein, and milk fractions, and that mare's milk is preferred.

7. A cosmetic according to one of the foregoing Claims, **characterized in that** it is the product of a direct and mild decomposition of a biological starting material with prior mechanical shearing treatment or ultrasonic treatment of the starting material, and of the subsequent condensation with sub-stoichiometric quantities of a fatty acid halide.

8. A cosmetic according to Claim 1, **characterized in that** the condensation product has a pH value from 5 to 7.

9. A cosmetic according to one of the foregoing Claims, **characterized in that** the cosmetic preparation is a cream, a body lotion, a hair washing agent, a hair conditioner, a mask, or a gel.

10. A cosmetic according to one of the foregoing Claims, **characterized in that** it is the product of a direct and mild decomposition of a biological starting material with subsequent condensation with sub-stoichiometric quantities of a fatty acid halide and subsequent carboxymethylisation or succinylisation.

11. A process for the manufacture of a cosmetic from condensed decomposition products of plant and animal origin, **characterized in that** a biological starting material, selected rom the group which consists of yeasts, yeast fractions, pulses, pulse fractions, pectins, pectin-containing masses, algae, algae fractions, animal milk, animal milk fractions, and their mixtures, is decomposed in a weakly alkaline medium in the pH range from 7.5 to 8.5 in the temperature range from 20 to 55°C, if applicable after prior mechanical shear treatment or ultrasonic treatment, and is condensed with a sub-stoichiometric quantity of a halide of C₁₀-C₂₀ fatty acid or a fatty acid halide mixture of such a fatty acid, after which the pH value is adjusted to values in the range from 5 to 7, and the product obtained is homogenised without further separation, and, if applicable, the homogenisate is mixed with other cosmetic substances from the group of carrier substances, ancillary substances, and active substances, and processed to a cosmetic preparation, in which situation the temperature for the biological decomposition product does not exceed 55°C and the pH value of the final product is between pH 5 and 7.

12. A process according to Claim 11, **characterized in that** the adjustment of the pH value in the weakly alkaline range is effected with an alkali hydroxide or alkali carbonate.

13. A process according to Claim 11, **characterized in that** the adjustment of the pH value in the weakly acidic range is effected with an acid, which is selected from the group which consists of hydrochloric acid, tannic acid, malic acid, and citric acid.

14. A process according to Claim 11, **characterized in that** the biological starting material is homogenised and partially decomposed by the effect of ultrasonics or by mechanical shearing forces or both, in which situation the temperature during decomposition does not exceed 55°C and the pH value lies in the range from 5 to 8.

15. A process according to one of the foregoing Claims, **characterized in that** the biological decomposition product is esterised or etherised after conversion with the fatty acid halide.

16. A process according to Claim 15, **characterized in that** the biological decomposition product is carboxymethylised with monochloroacetic acid or dichloroacetic acid, or succinylised with succinic acid hydride.

17. A process according to one of the foregoing Claims, **characterized in that** a quantity of hydrogen peroxide is added to the biological decomposition product for further decolorisation or odour binding, which is to be converted in the maximum quantity.

18. The use of a cosmetic from condensed decomposition products of plant and animal origin according to Claim 1, as a cosmetic raw material.

19. The use of a cosmetic from condensed decomposition products of plant and animal origin according to Claim 1, for the manufacture of cosmetic preparations together with other cosmetic ancillary, carrier, and/or active substances.

## Revendications

1. Produit cosmétique obtenu à partir de produits de dégradation d'origine végétale et animale condensé, **caractérisé en ce qu'**il s'agit d'un produit d'une dégradation directe et modérée (20 à 55°C ; pH 7,5 à 8,5) d'un matériau de départ biologique dans un milieu aqueux, suivie d'une condensation avec une quantité sous-stoechiométrique d'un halogénure d'acide gras en C10 à C20 ou d'un mélange d'halogénures d'acides gras en C10 à C20 et le cas échéant suivie d'une éthérification ou estérification du produit de condensation,
le matériau de départ étant choisi dans le groupe comprenant des levures, des fractions de levures, des légumineuses, des fractions de légumineuses, des pectines, des substances contenant de la pectine, des algues, des fractions d'algues, du lait d'animaux, des fractions de lait d'animaux et des mélanges de ces substances,
et le produit de dégradation condensé contenant des structures biologiques du matériau de départ choisies dans le groupe comprenant des composants de substances naturelles à structure en hélice, des structures enzymatiques et des structures vitaminiques,
et le produit de condensation étant le cas échéant présent dans une préparation cosmétique avec d'autres adjuvants, véhicules ou principes actifs cosmétiques.

2. Produit cosmétique selon la revendication 1, **caractérisé en ce que** le matériau de départ biologique est une levure ou une fraction de levure choisie dans le groupe comprenant la levure de boulanger et la levure de bière.

3. Produit cosmétique selon la revendication 1, **caractérisé en ce que** le matériau de départ biologique est une légumineuse ou une fraction de légumineuse choisie dans le groupe comprenant les pois, les lentilles, le soja et la féverole.

4. Produit cosmétique selon la revendication 1, **caractérisé en ce que** le matériau de départ biologique est une pectine ou une substance contenant de la pectine choisie dans le groupe comprenant les écales d'agrumes, le marc de pomme et le marc de raisin.

5. Produit cosmétique selon la revendication 1, **caractérisé en ce que** le matériau de départ biologique est une algue choisie dans le groupe comprenant des algues vertes, des algues brunes, des algues rouges et des mélanges de celles-ci avec des proportions élevées de chlorophylle, d'alginates, de protéines et de substances minérales.

6. Produit cosmétique selon la revendication 1, **caractérisé en ce que** le matériau de départ biologique est du lait d'animaux choisi dans le groupe comprenant le lait de jument, le lait de vache, le lait de brebis, le lait de renne, le lait de chèvre, des protéines de petit-lait et des fractions du lait, et **en ce que** le lait de jument est préféré.

7. Produit cosmétique selon l'une des revendications précédentes, **caractérisé en ce qu'**il s'agit du produit d'une dégradation directe et modérée d'un matériau de départ biologique, traité au préalable par un traitement de cisaillement mécanique ou un traitement aux ultrasons du matériau de départ, et d'une condensation ultérieure avec des quantités sous-stoechiométriques d'un halogénure d'acide gras.

8. Produit cosmétique selon la revendication 1, **caractérisé en ce que** le produit de condensation a un pH de 5 à 7.

9. Produit cosmétique selon l'une des revendications précédentes, **caractérisé en ce que** la préparation cosmétique est une crème, une lotion de toilette corporelle, un shampooing, un après-shampooing, un masque ou un gel.

10. Produit cosmétique selon l'une des revendications précédentes, **caractérisé en ce qu'**il s'agit du produit d'une dégradation directe et modérée d'un matériau de départ biologique, suivie d'une condensation avec des quantités sous-stoechiométriques d'un halogénure d'acide gras et d'une carboxy-méthylation ou succinylation ultérieure.

11. Procédé de préparation d'un produit cosmétique à partir de produits de dégradation d'origine végétale et animale condensés, **caractérisé en ce que** l'on dégrade un matériau de départ biologique choisi dans le groupe comprenant des levures, des fractions de levures, des légumineuses, des fractions de légumineuses, des pectines, des substances contenant de la pectine, des algues, des fractions d'algues, du lait d'animaux et des fractions de lait d'animaux ainsi que des mélanges de ces substances, dans un milieu aqueux faiblement basique, avec un pH dans la plage de 7,5 à 8,5, dans une plage de température de 20 à 55°C et on le condense avec une quantité sous-stoechiométrique d'un halogénure d'un acide gras en C10 à C20 ou d'un mélange d'halogénures de ces acides gras, ensuite on ajuste le pH à des valeurs dans la plage de 5 à 7, et le produit obtenu, qui contient des structures biologiques du produit de départ choisies dans le groupe comprenant des composants de substances naturelles à structure en hélice, des structures enzymatiques et des structures vitaminiques et des mélanges de celles-ci, est homogénéisé sans autre séparation et l'homogénéisat est le cas échéant mélangé avec d'autres substances cosmétiques du groupe des véhicules, des adjuvants et des principes actifs et le cas échéant transformé en une préparation cosmétique, la température ne dépassant pas 55°C pour le produit de dégradation biologique et le pH du produit final se situant entre pH 5 et pH 7.

12. Procédé selon la revendication 11, **caractérisé en ce que** l'ajustement du pH dans le domaine faiblement basique se fait avec un hydroxyde de métal alcalin ou un carbonate de métal alcalin.

13. Procédé selon la revendication 11, **caractérisé en ce que** l'ajustement du pH dans le domaine faiblement acide se fait avec un acide choisi dans le groupe comprenant l'acide chlorhydrique, l'acide tartrique, l'acide malique et l'acide citrique.

14. Procédé selon la revendication 11, **caractérisé en ce que** le matériau de départ biologique est homogénéisé par l'action d'ultrasons ou de forces de cisaillement mécanique ou les deux et partiellement dissocié, la température au cours de la désagrégation ne dépassant pas 55°C et le pH se situant dans la plage de 5 à 8.

15. Procédé selon l'une des revendications précédentes, **caractérisé en ce que** le produit de dégradation biologique est estérifié ou éthérifié après la réaction avec l'halogénure d'acide gras.

16. Procédé selon la revendication 15, **caractérisé en ce que** le produit de dégradation biologique est carboxyméthylé avec de l'acide mono- ou dichloracétique ou succinylé avec de l'anhydride succinique.

17. Procédé selon l'une des revendications précédentes, **caractérisé en ce que** l'on ajoute au produit de dégradation biologique, pour le décolorer davantage ou supprimer l'odeur, une quantité de peroxyde d'hydrogène qui au maximum doit réagir quantitativement.

18. Utilisation d'un produit cosmétique obtenu à partir de produits de dégradation d'origine végétale et animale condensés selon la revendication 1 comme substance de base cosmétique.

19. Utilisation d'un produit cosmétique obtenu à partir de produits de dégradation d'origine végétale et animale condensés selon la revendication 1 pour produire des préparations cosmétiques ensemble avec d'autres adjuvants, véhicules et/ou principes actifs cosmétiques.
